# EUROPEAN PATENT APPLICATION

(11) **EP 3 162 406 A1**
(43) Date of publication of application: **03.05.2017**
(21) Application number: 15811194.8
(22) Date of filing: 16.06.2015
(51) Int. Cl.: A61M 25/10

(54) **BODY FLUID FLOW NON-BLOCKING BALLOON CATHETER**

(30) Priority: 25.06.2014 JP 2014129798
(71) Applicant: Fumiyama, Hideo, Tokyo 135-0021 (JP)
(72) Inventor: Fumiyama, Hideo, Tokyo 135-0021 (JP)
(74) Representative: Dr. Gassner & Partner mbB
(86) International application number: PCT/JP2015/067312
(87) International publication number: WO 2015/198918

(57) **Abstract**

The present invention provides a low-profile balloon catheter capable of providing pressure-contact to a wall of a lumen or a stent or a stent graft in the lumen without causing flow of body fluid such as blood to be interrupted when the balloon is expanded in the lumen. The present balloon catheter 1 includes a single balloon 3 arranged at a distal side of a catheter shaft 2, and three line-shaped portions 11 extending outside the balloon over the entire length of the balloon along an axial direction of the catheter shaft as being arranged at even intervals in a circumferential direction. The balloon is partially restricted from being expanded by the line-shaped portions and is expanded in a radial direction between the adjacent line-shaped portions to be formed into three balloon expansion portions 3a to 3c that are divided in the circumferential direction. Accordingly, gaps 14a to 14c are formed between a lumen wall 13 and outer circumferential faces of the balloon expansion portions so that blood or body fluid can be flown therethrough without being interrupted.

## Description

### TECHNICAL FIELD

The present invention relates to a body fluid flow non-interrupting type balloon catheter to be used as being inserted into a lumen of a human or another animal.

### BACKGROUND ART

In a recent medical treatment, less-invasive transcatheter that causes a relatively small burden to a patient has been popularly performed. For example, in a treatment of aortic aneurysm or aortic dissection, a body fluid flow non-interrupting type balloon catheter has been used to adapt a stent graft indwelling in a blood vessel to a blood vessel inner wall at a desired position. Further, transdermal angioplasty and transdermal valvuloplasty to expand a narrowed blood vessel or a narrowed valve with a balloon catheter have been performed in a circulating system treatment such as a subaortic stenosis.

Since a balloon included in a normal balloon catheter interrupts blood flow while the balloon is expanded in a blood vessel, long-term expansion may cause a dangerous case for a patient. Particularly, with transdermal valvuloplasty, there may be a risk that long-term blood flow interruption causes concomitant diseases such as blood pressure drop, unconsciousness, and cardiogenic shock due to myocardial ischemia. Further, there may be a risk that a stent graft causes migration due to blood flow ejected from a heart when the balloon is expanded.

In view of the above, a variety of blood flow non-interrupting type balloon catheters devised not to interrupt blood flow during expansion of a balloon have been developed and used (e.g., see Patent Documents 1 and 2 and Non-Patent Document 1). According to a body fluid flow non-interrupting type balloon catheter disclosed in Patent Document 1, a plurality of convex portions are formed radially in a cross-section in a direction perpendicular to a catheter axis of a balloon. Here, when the balloon is expanded, the convex portions of the balloon expand a blood vessel, so that blood flows through concave portions of the balloon.

In balloon catheters disclosed in Patent Document 2 and Non-Patent Document 1, a plurality of separated balloons are arranged at a leading end of a catheter at even intervals in a circumferential direction. In the balloon catheter, each balloon is attached to the catheter at both ends thereof. An expanding solution is supplied to the respective balloons as being branched from a passage in the catheter, so that the balloons are concurrently expanded. Here, blood flows through a gap formed between the adjacent balloons.

### CITED DOCUMENT

### PATENT DOCUMENT

Patent Document 1: Japanese Patent Application Laid-Open No. 8-112351
Patent Document 2: Japanese Patent Application Laid-Open No. 2012-505050

### NON-PATENT DOCUMENT

Non-Patent Document 1: "Goatrilobe balloon catheter II", Revised on October 15, 2013 (Forth edition), Attached document information of medical instrument (search page), Pharmaceuticals and Medical Devices Agency (Searched on H26.06,04), Internet <URL: http://www.info.pmda.go.jp/ygo/ pack/22200BZX00729000_A_04_01/>

### SUMMARY OF THE INVENTION

Since the balloon catheter disclosed in Patent Document 1 maintains a shape (e.g., a star-shaped cross-section) enabling blood to pass between the convex portions and a blood vessel wall while the convex portions expand the blood vessel during balloon expansion, considerable degree of stiffness is required therefor. Accordingly, the balloon becomes less flexible at least than usual in both expanding and contracting directions to be incapable of being folded into a low profile along an outer circumference of the catheter. Further, a part of the catheter where the balloon is attached is difficult to be largely bent. As a result, it is extremely difficult to pass through a curved portion in a lumen or a slim lumen, so that a transcatheter treatment is largely restricted.

According to the balloon catheters disclosed in Patent Document 2 and Non-Patent Document 1, three or more balloons are arranged in the circumferential direction. Therefore, even if all of the balloons are flexibly contracted, an outer diameter of the whole becomes large compared to a case of a normal balloon, that is, a single balloon. Consequently, in a case of a patient having a slim blood vessel or a slim lumen, the catheter cannot be inserted thereinto. For enabling the insertion, a catheter insertion hole to be arranged at a blood vessel is required to be opened large. As a result, transcatheter treatment is restricted.

Recently, a stent graft having a smaller diameter has been used for indwelling in a slim lumen. In such a case, it is required to prepare a body fluid flow non-interrupting type balloon catheter that provides small-diameter blood flow corresponding to a size of the stent graft for pressure-contacting the stent graft to a blood vessel wall in a stent-grafting treatment or in a treatment for end leakage occurring thereafter.

On the other hand, there has been conventionally used, to ensure blood flow even during balloon expansion, a perfusion catheter in which a lumen for blood bypassing and a hole opened into a blood vessel are arranged at parts of a catheter shaft being front and rear from a balloon. However, normally, since sectional area of the lumen and a dimension of the hole are small, a blood flow amount is relatively small. For increasing the blood flow amount, the catheter shaft is required to be thickened. Thus, application to a transcatheter treatment into a slim lumen is restricted.

In view of the above, an object of the present invention is to provide a body fluid flow non-interrupting type balloon catheter that provides a low-profile blood flow and that is capable of providing pressure-contact to a wall of a lumen or device such as a stent or a stent graft in the lumen without causing flow of body fluid such as blood in the lumen when the balloon is expanded and capable of being easily inserted into a slimmer lumen.

The present invention provides a body fluid non-interrupting type balloon catheter to be inserted into a lumen of a living body or a device in the lumen including a catheter shaft, a single balloon arranged at a distal side of the catheter shaft, and a plurality of linear line-shaped portions extending outside the balloon over the entire length of the balloon along an axial direction of the catheter shaft as being arranged at predetermined intervals in a circumferential direction. Here, the balloon forms a plurality of balloon expansion portions as being expanded in a radial direction between the adjacent line-shaped portions and divided in the circumferential direction by the line-shaped portions. Further, the line-shaped portions restrict expansion of the balloon so that a gap having a size to be capable of flowing body fluid between a distal side and a base end side of the balloon is formed between the lumen or an inner wall of the device and the adjacent balloon expansion portion.

In the present invention, owing to that the plurality of line-shaped portions are arranged, the single balloon is expanded in the radial direction between the adjacent line-shaped portions to be formed into the plurality of balloon expansion portions divided in the circumferential direction. Accordingly, the gaps are formed between outer circumferential faces of the balloon expansion portions and a lumen wall or an inner wall of a stent or a stent graft so that body fluid such as blood can be flown through the gaps without being interrupted. Accordingly to the above, in a transcatheter treatment, it is possible to effectively avoid risks that may occur due to interruption of blood flow or the like in a lumen, for example, concomitant diseases such as blood pressure drop, unconsciousness, and cardiogenic shock due to myocardial ischemia in transdermal valvuloplasty.

Further, since the present invention includes only the single balloon, it is possible to be folded small when being contracted. Consequently, it is possible for the balloon catheter of a blood flow non-interrupting type and a body fluid flow non-interrupting type to have a low profile. Accordingly, it is possible to perform a less-invasive transcatheter treatment by enabling insertion into a slim lumen or lessening an insertion hole to a lumen.

In an embodiment of the balloon catheter of the present invention, the plurality of line-shaped portions are three line-shaped portions. Accordingly, the three balloon expansion portions are formed respectively between the adjacent line-shaped portions. According to the above, it is possible to have appropriate setting in a balanced manner for causing expansion force necessary for sufficiently pressure-contacting the balloon to a lumen wall or the device such as a stent and a stent graft in a lumen and forming sufficiently large gaps between the above and the balloon expansion portions so as not to interrupt blood flow and the like.

In another embodiment, the plurality of line-shaped portions are arranged at even intervals in the circumferential direction. Accordingly, the plurality of balloon expansion portions being adjacent in the circumferential direction are formed to be equal in size and to be into the approximately same shape. According to the above, pressure-contacting force to a lumen wall or the device such as a stent or a stent graft in a lumen can be distributed evenly in the circumferential direction. In addition, gaps between outer circumferential faces of the balloon expansion portions and the lumen wall or the inner wall of the stent, the stent graft, or the like can be formed evenly in the circumferential direction.

In a certain embodiment, both ends of the plurality of line-shaped portions are fixed to a pair of attachment members that are attached to the catheter shaft as being mutually distanced in the axial direction. Accordingly, the body fluid flow non-interrupting type balloon catheter of the present invention can be easily manufactured by using a conventional body fluid flow non-interrupting type balloon catheter.

Here, in another embodiment, the plurality of line-shaped portions and the pair of attachment members may be integrally formed. Accordingly, the body fluid flow non-interrupting type balloon catheter can be manufactured more easily.

Further, in another embodiment, at least either of the pair of attachment members is a radiopaque marker. In such a body fluid flow non-interrupting type balloon catheter, it is commonly performed to attach a radiopaque marker for recognizing a position of a balloon in a lumen. Here, owing to that the attachment member of the plurality of line-shaped portions functions as the radiopaque marker as well, the structure of the body fluid flow non-interrupting type balloon catheter can be simplified and parts count thereof can be reduced.

### BRIEF DESCRIPTION OF THE DRAWINGS

FIG. 1 is a partial side view illustrating a balloon in an expanded state in a preferable embodiment of a body fluid flow non-interrupting type balloon catheter (hereinafter, simply called a balloon catheter) of the present invention.
FIG. 2 is a partial side view illustrating the balloon in a contracted state.
FIG. 3 is a sectional view at line III-III in FIG. 1.
FIG. 4 is a sectional view at line IV-IV in FIG. 1.
FIG. 5 is a side view of a balloon expansion restricting unit in FIG. 1.
FIG. 6 is a side view of another embodiment of the balloon expansion restricting unit.

### EMBODIMENTS OF THE INVENTION

In the following, a balloon catheter of the present invention will be described in detail based on preferable embodiments with reference to the attached drawings.

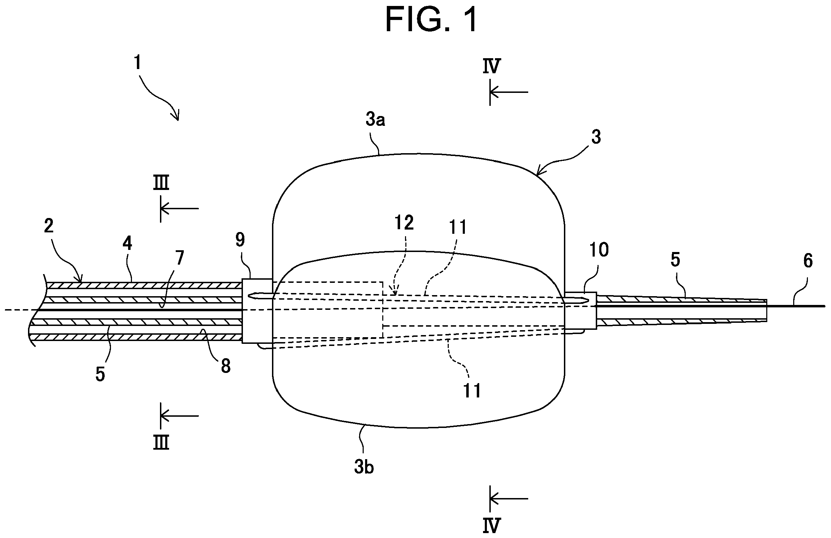

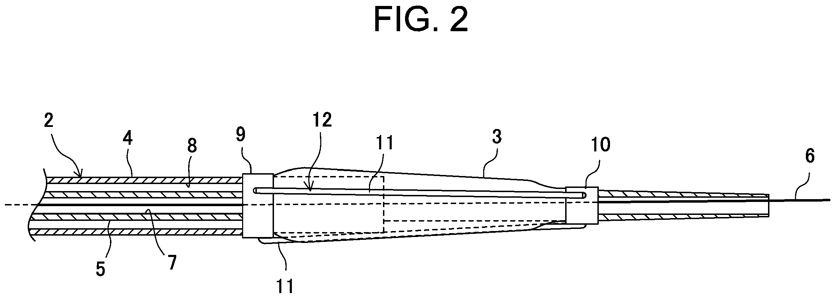

FIGs. 1 and 2 illustrate an enlarged main part of a balloon catheter of an embodiment. The balloon catheter 1 of the present embodiment includes a catheter shaft 2 and a balloon 3 arranged at a distal side (i.e., a leading end part in the present embodiment).

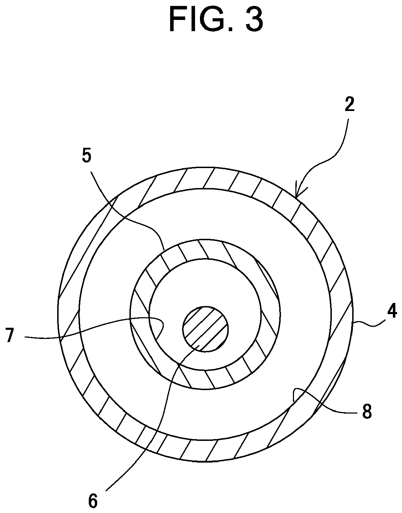

The catheter shaft 2 includes an outer shaft 4 and an inner shaft 5 that is inserted into the inside of the outer shaft 4 and protruded from a leading end thereof by a predetermined length. As illustrated in FIG. 3, an inner lumen 7 for having a guide wire 6 be inserted therein is defined at the inside of the inner shaft 5. An outer lumen 8 for feeding a balloon expanding medium as described later is defined at the inside of the outer shaft 4 between an inner face of the outer shaft and an outer face of the inner shaft 5.

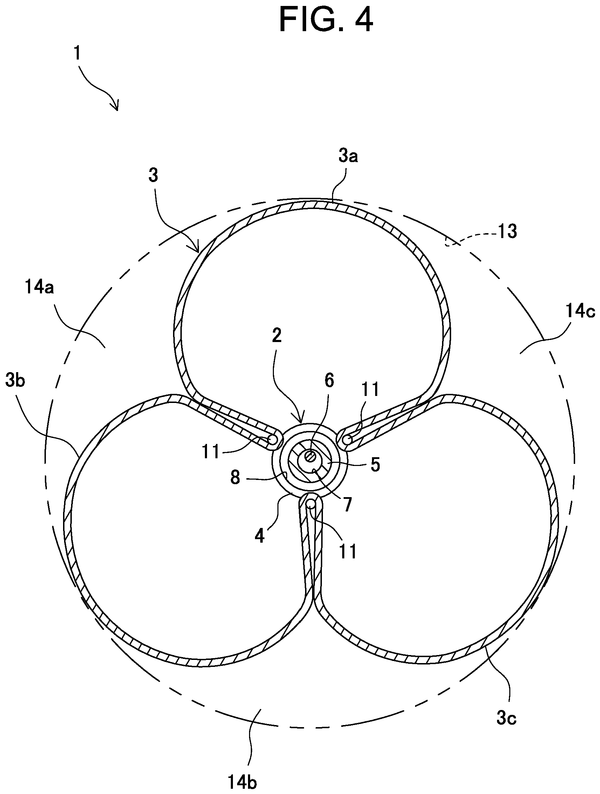

The balloon 3 is capable of being folded at an outer circumferential face of the catheter shaft 2. FIG. 1 illustrates an expanded state of the balloon 3 and FIG. 2 illustrates a contracted state of the balloon 3. A proximal end of the balloon 3 is fixed to an outer circumferential face of the outer shaft 4 in a liquid-tight manner at a position slightly shifted to the proximal side from the leading end of the outer shaft 4. A distal end thereof is fixed to the outer circumferential face of the inner shaft 5 in a liquid-tight manner at a position slightly shifted to the proximal side from the leading end of the inner shaft 5. As illustrated in FIG. 4, inner space of the balloon 3 is in communication with the outer lumen 8 to be capable of introducing or discharging a balloon expanding medium.

A pair of radiopaque markers 9, 10 at a proximal side and a distal side are arranged at the catheter shaft 2 as being mutually distanced in the axial direction so that a position of the balloon 3 can be easily recognized. The proximal side marker 9 is arranged to cover the proximal end of the balloon 3 and the distal side marker 10 is arranged to cover the distal end of the balloon 3.

A balloon expansion restriction unit 12 having three line-shaped portions 11 extending at least over the entire length of the balloon 3 straightly along the axial direction of the catheter shaft 2 is arranged at the outside of the balloon 3. The line-shaped portions 11 are arranged at even intervals in the circumferential direction. Both ends of each line-shaped portion 11 are fixed to the proximal side marker 9 and the distal side marker 10.

During a treatment, a predetermined amount of balloon expanding medium is supplied to the inner space of the balloon from a base end of the catheter shaft 2 through the outer lumen 8 as being similar to the prior art to expand the balloon 3 that is delivered to a predetermined position in a lumen. Since the balloon 3 is partially restricted from being expanded in a radial direction by the three line-shaped portions 11, the balloon 3 is expanded as being divided into three balloon expansion portions 3a to 3c in the circumferential direction between the adjacent line-shaped portions, as illustrated in FIG. 4.

According to the above, gaps 14a to 14c are formed between a lumen wall (or an inner wall of a stent or a stent graft) 13 where the balloon 3 is located and outer circumferential faces of the balloon expansion portions 3a to 3c. The gaps 14a to 14c have sufficient cross-sectional area for body fluid such as blood to flow in the lumen without being interrupted. Thus, according to a transcatheter treatment using the balloon catheter 1 of the present invention, it is possible to effectively avoid risks that may occur due to interruption of blood flow or the like in a lumen, for example, concomitant diseases such as blood pressure drop, unconsciousness, and cardiogenic shock due to myocardial ischemia in transdermal valvuloplasty.

The line-shaped portions 11 of the present embodiment are formed of, for example, metal-made wire having high tension strength. Other than metal, it is possible to adopt, as a material for the line-shaped portions 11, a plastic material that is strengthened with carbon fibers, glass fibers, or the like. The balloon 3 is restricted to be expanded in the radial direction by the line-shaped portions 11 to be expanded as being divided in the circumferential direction. As long as a sufficiently large gap can be formed against a lumen wall or the like due to the above, the line-shaped portions 11 may be formed of materials having a variety of material qualities, mechanical properties such as tension strength and stiffness, and characteristics and/or may be formed into a variety of shapes.

The balloon catheter 1 of the present invention is required to have appropriate setting in a balanced manner for causing expansion force necessary for sufficiently pressure-contacting the balloon 3 to a lumen wall 13 or an inner wall of a stent, a stent graft, or the like in a lumen and forming sufficiently large gaps between the walls and the balloon expansion portions 3a to 3c so as not to interrupt blood flow and the like. In the present embodiment, the above is actualized by arranging the three line-shaped portions. However, in another embodiment, it is also possible to arrange four or more line-shaped portions depending on usage and use conditions.

Further, in the present embodiment, owing to that the line-shaped portions 11 are arranged at even intervals in the circumferential direction, the balloon expansion portions 3a to 3c are formed to be equal in size and to be into the approximately same shape. Accordingly, pressure-contacting force to a lumen wall or an inner wall of a stent, a stent graft, or the like can be distributed evenly in the circumferential direction. In addition, gaps between outer circumferential faces of the balloon expansion portions and the lumen wall or the inner wall of the stent, the stent graft, or the like can be formed evenly in the circumferential direction.

After a treatment, the balloon expanding medium is sucked from the base end of the catheter shaft 2 through the outer lumen 8 to be eliminated from the inner space of the balloon 3. Thus, the balloon 3 is returned into the original contracted state illustrated in FIG. 2. Since only one balloon is arranged in the balloon catheter 1 of the present invention, the balloon can be folded into a low profile to be small along the outer circumferential face of the catheter shaft 2. Accordingly, it is possible to perform a less-invasive transcatheter treatment by enabling insertion into a slim lumen or lessening an insertion hole to a lumen.

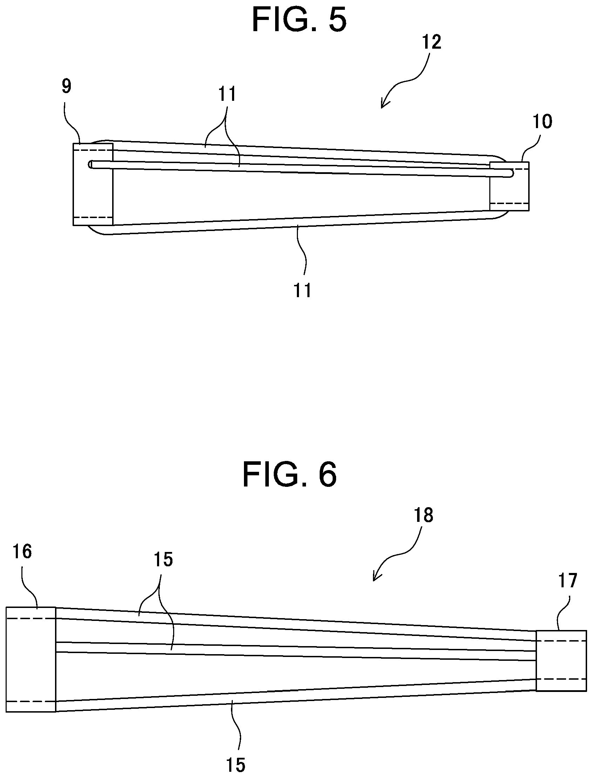

In the present embodiment, as illustrated in FIG. 5, the balloon expansion restricting unit 12 can be structured as a single component including the three line-shaped portions 11 and the pair of markers 9, 10 integrally fixed to both ends thereof. Accordingly, the structure of the balloon catheter can be simplified and parts count thereof can be reduced. Further, in a case that the balloon expansion restricting unit 12 being a single component is attached to a conventional balloon catheter, the balloon catheter of the present invention can be easily manufactured.

In another embodiment, it is possible that either or both of the markers 9, 10 can be structured simply as a non-radiopaque attachment member to a catheter shaft. In another embodiment, as illustrated in FIG. 6, an entire balloon expansion restricting unit 18 including three line-shaped portions 15 and a pair of attachment members 16, 17 that fixes both ends thereof can be integrally formed of for example, the abovementioned strengthened plastic material. Accordingly, the balloon catheter of the present invention can be manufactured more easily.

In the above, the body fluid flow non-interrupting type balloon catheter of the present invention is described in detail based on preferable embodiments. Here, in the technical scope thereof, the present invention can be actualized while applying various modifications and transformations to the abovementioned embodiments.

### EXPLANATION OF REFERENCES

- 1: Body fluid flow non-interrupting type balloon catheter
- 2: Catheter shaft
- 3: Balloon
- 4: Outer shaft
- 5: Inner shaft
- 6: Guide wire
- 7: Inner lumen
- 8: Outer lumen
- 9, 10: Marker
- 11: Line-shaped portion
- 12: Balloon expansion restricting unit

## Claims

1. A body fluid non-interrupting type balloon catheter to be inserted into a lumen of a living body or a device in the lumen, comprising:
a catheter shaft;
a single balloon arranged at a distal side of the catheter shaft; and
a plurality of linear line-shaped portions extending outside the balloon over the entire length of the balloon along an axial direction of the catheter shaft as being arranged at predetermined intervals in a circumferential direction,
wherein the balloon forms a plurality of balloon expansion portions as being expanded in a radial direction between the adjacent line-shaped portions and divided in the circumferential direction by the line-shaped portions, and
the line-shaped portions restricts expansion of the balloon so that a gap having a size to be capable of flowing body fluid between a distal side and a base end side of the balloon is formed between the lumen or an inner wall of the device and the adjacent balloon expansion portion.

2. The body fluid non-interrupting type balloon catheter according to claim 1, wherein the plurality of line-shaped portions are three line-shaped portions.

3. The body fluid non-interrupting type balloon catheter according to claim 1 or claim 2, wherein the plurality of line-shaped portions are arranged at even intervals in the circumferential direction.

4. The body fluid non-interrupting type balloon catheter according to any one of claims 1 to 3, wherein both ends of the plurality of line-shaped portions are fixed to a pair of attachment members that are attached to the catheter shaft as being mutually distanced in the axial direction.

5. The body fluid non-interrupting type balloon catheter according to claim 4, wherein the plurality of line-shaped portions and the pair of attachment members are integrally formed.

6. The body fluid non-interrupting type balloon catheter according to claim 4 or claim 5, wherein at least either of the pair of attachment members is a radiopaque marker.
